(19) Europäisches Patentamt
European Patent Office
Office européen des brevets

(11) **EP 2 570 798 A1**

(12) **EUROPEAN PATENT APPLICATION**
published in accordance with Art. 153(4) EPC

(43) Date of publication:
**20.03.2013 Bulletin 2013/12**

(51) Int Cl.:
**G01N 21/64** (2006.01)  **G01N 33/542** (2006.01)
**G01N 33/566** (2006.01)

(21) Application number: **11780363.5**

(22) Date of filing: **06.05.2011**

(86) International application number:
**PCT/JP2011/002546**

(87) International publication number:
**WO 2011/142103 (17.11.2011 Gazette 2011/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.05.2010 JP 2010110383**

(71) Applicant: **Mitsui Engineering & Shipbuilding Co.,
Ltd.**
**Chuo-ku**
**Tokyo 104-8439 (JP)**

(72) Inventors:
• **HOSHISHIMA, Kazuteru**
**Tamano-shi**
**Okayama 706-8651 (JP)**
• **NAKADA, Shigeyuki**
**Tamano-shi**
**Okayama 706-8651 (JP)**

(74) Representative: **Hards, Andrew**
**Global IP Europe**
**Patentanwaltskanzlei**
**Pfarrstrasse 14**
**80538 München (DE)**

(54) **FRET MEASUREMENT METHOD AND FRET MEASUREMENT DEVICE**

(57) Disclosed herein is a method for measuring FRET by irradiating with laser light a measurement sample. FRET is transfer of energy from a first molecule to a second molecule. The first molecule and the second molecule are included in the measurement sample in which ligands are bound to receptors. The method includes the steps of: irradiating the measurement sample with laser light; measuring fluorescence emitted by the measurement sample; calculating a fluorescence lifetime of the first molecule; calculating a binding ratio; setting a binding condition for the measurement sample; and calculating a dissociation constant. In the dissociation constant calculating step, the dissociation constant is determined by using a least-squares method to fit a function having, as variables, a total concentration of the receptor in the measurement sample and the dissociation constant to the binding ratio calculated in the binding ratio calculating step.

FIG.6

EP 2 570 798 A1

**Description**

Technical Field

The present invention relates to a method and a device for measuring fluorescence resonance energy transfer (FRET) that is the transfer of energy from a donor molecule (first molecule) that absorbs energy by irradiation with laser light to an acceptor molecule (second molecule). More specifically, the present invention relates to a FRET measurement device and a FRET measurement method for determining a dissociation constant by irradiating with laser light a measurement sample in which a receptor is bound to a ligand and a donor molecule and an acceptor molecule are provided.

Background Art

Analysis of protein functions has recently become important as post-genome-related technology in medical care, drug discovery, and food industry. Particularly, it is necessary to research interactions (association and dissociation) between a protein which is a living substance in a living cell and another protein or a low-molecular compound, to analyze the actions of cells.

A fluorescence resonance energy transfer (FRET) phenomenon is utilized to analyze interactions between a protein which is a living substance in a living cell and another protein or a low-molecular compound. By measuring fluorescence generated by a FRET phenomenon, interactions between molecules in a range of several nanometers can be measured.

For example, a technique is known which determines FRET efficiency, which indicates the degree of energy transfer from a donor molecule to an acceptor molecule, by using a fluorescence lifetime $\tau^*_d$ of the donor molecule at the time of occurrence of FRET and a fluorescence lifetime $\tau_d$ of the donor molecule in the absence of the acceptor molecule (Patent Literature 1).

In the above-described Patent Literature 1, the FRET efficiency is determined by $1-\tau^*_d/\tau_d$.

However, the above-described FRET efficiency is influenced by the concentration of the donor or acceptor molecules, and therefore it is difficult to quantitatively determine the strength of interaction between proteins contained in cells or the like by using the above-described technique.

A dissociation constant $K_d$ is generally used as an index indicating the strength of binding between molecules interacting with each other. However, there is a case where it is not easy to accurately measure a dissociation constant $K_d$. Hereinbelow, a dissociation constant $K_d$ will be described. For example, in the case of a reaction represented by the following formula (1), a dissociation constant $K_d$ is defined as the following formula (2). Here, L represents a ligand, R represents a receptor, and LR represents the receptor bound to the ligand. Further, [L] represents the concentration of the ligand not bound to the receptor (hereinafter, referred to as a "ligand concentration"), [R] represents the concentration of the receptor not bound to the ligand (hereinafter, referred to as a "receptor concentration"), and [LR] represents the concentration of the receptor bound to the ligand.

[Formula 1]

$$L + R \iff LR \qquad (1)$$

[Formula 2]

$$K_d = \frac{[L][R]}{[LR]} \qquad (2)$$

When the total concentrations of the ligand and the receptor in a sample are defined as $[L_0]$ and $[R_0]$, respectively, $[L_0]$ and $[R_0]$ are represented by the following relational expressions:

[Formula 3]

$$[L_0] = [L] + [LR] \qquad (3)$$
$$[R_0] = [R] + [LR] \qquad (4)$$

The following formula (5) is obtained by eliminating [R] from the formulas (2) and (4):

[Formula 4]

$$\frac{[LR]}{[R_0]} = \frac{[L]}{K_d + [L]} \qquad (5)$$

The left member of the formula (5) represents the ratio of the receptors bound to the ligands to the total receptors. When $[LR]/[R_0]$ is plotted against the logarithmic axis of [L], a sigmoid curve is obtained.

A larger value of the dissociation constant $K_d$ indicates that the reaction represented by the formula (1) is more likely to proceed from the right to the left. Therefore, when the dissociation constant $K_d$ is large to some extent, the relationship [L] » [LR] is established. In this case, $[LR]/[R_0]$ is approximated as given in the following formula (6) based on the formulas (3) and (5):

[Formula 5]

$$\frac{[LR]}{[R_0]} \approx \frac{[L_0]}{K_d + [L_0]} \qquad (6)$$

According to the formula (6), when the total ligand concentration $[L_0]$ is equal to the dissociation constant $K_d$, the ratio of the receptors bound to the ligands to the total receptors contained in the sample, that is, $[LR]/[R_0]$ becomes 0.5. Therefore, the dissociation constant $K_d$ can be determined from the total ligand concentration $[L_0]$ at which $[LR]/[R_0]$ measured by changing the total ligand concentration $[L_0]$ becomes 0.5.

Even when it is difficult to measure the concentration [L] of the ligand at equilibrium, the total ligand concentration $[L_0]$ can be determined. For this reason, the above-described method is used to determine the dissociation constant $K_d$.

Citation List

Patent Literature

Patent Literature 1: Japanese Patent Application Laid-Open No. 2007-240424

Summary of Invention

Technical Problem

However, when the dissociation constant $K_d$ is small, the relational expression [L] » [LR] is not established, and therefore the formula (5) cannot be approximated as the formula (6). Therefore, when the dissociation constant $K_d$ is small, the value of the total ligand concentration $[L_0]$ at which $[LR]/[R_0]$ measured by changing the total ligand concentration $[L_0]$ becomes 0.5 is different from the value of the dissociation constant $K_d$. Thus, the dissociation constant $K_d$ cannot be correctly determined by such a conventional method.

It is therefore an object of the present invention to provide a FRET measurement method and a FRET measurement device which are capable of accurately measuring a dissociation constant $K_d$ irrespective of the magnitude of the dissociation constant $K_d$.

A FRET measurement method of the present invention is a FRET measurement method for measuring, by irradiating a measurement sample with laser light, fluorescence resonance energy transfer (FRET) that is transfer of energy from a first molecule to a second molecule, the first molecule and the second molecule included in the measurement sample in which ligands are bound to receptors, the method including the steps of:

irradiating the measurement sample with laser light whose intensity is time-modulated;
measuring fluorescence emitted by the measurement sample irradiated with the laser light;
calculating a fluorescence lifetime of the first molecule by using a fluorescence signal measured in the measuring step;
calculating a binding ratio that is a ratio of FRET occurring receptors to the receptors contained in the measurement sample by using the fluorescence lifetime calculated in the fluorescence lifetime calculating step;
setting a binding condition for the measurement sample so that the binding ratio is changed; and
calculating a dissociation constant indicating a degree of binding between the receptors and the ligands,
wherein, in the dissociation constant calculating step, the dissociation constant is determined by using a least-squares method to fit a function to the binding ratio calculated in the binding ratio calculating step, the function having, as variables, a total concentration of the receptors in the measurement sample and the dissociation constant.

When the binding ratio, a total concentration of the receptors contained in the measurement sample, a total concentration of the ligands contained in the measurement sample, and the dissociation constant are defined as $\kappa_{FRET}$, $[R_0]$, $[L_0]$, and $K_d$, respectively, the dissociation constant $K_d$ is preferably determined in the dissociation constant calculating step by using a two-variable least-squares method using the $[R_0]$ and the $K_d$ as variables to fit the following formula to the $\kappa_{FRET}$ and the $[L_0]$:

[Formula 6]

$$\kappa_{FRET} = \frac{[L_0] + [R_0] + K_d - \sqrt{([L_0] + [R_0] + K_d)^2 - 4[L_0][R_0]}}{2[R_0]}$$

In the fluorescence lifetime calculating step, a fluorescence lifetime of the first molecule is preferably calculated using a phase difference between a fluorescence signal measured in the measuring step and a modulation signal that modulates the laser light.

Preferably, the first molecule and the second molecule are bound to one of the receptors, and, in the binding condition setting step, a binding condition for the measurement sample is preferably set by adding ligands to the measurement sample so that the binding ratio is changed.

Alternatively, it is preferable that the first molecule may be bound to one of the receptors, the second molecule is bound to one of the ligands, and, in the binding condition setting step, a binding condition for the measurement sample is set by adding ligands to the measurement sample so that the binding ratio is changed.

A FRET measurement device of the present invention is a FRET measurement device for measuring, by irradiating a measurement sample with laser light, fluorescence resonance energy transfer (FRET) that is transfer of energy from a first molecule to a second molecule, the first molecule and the second molecule included in the measurement sample in which ligands are bound to receptors, the device including:

a laser light source unit operable to irradiate the measurement sample with laser light whose intensity is time-modulated;
a measurement unit operable to measure fluorescence emitted by the measurement sample irradiated with the laser light;
a fluorescence lifetime calculation unit operable to calculate a fluorescence lifetime of the first molecule by using a fluorescence signal measured by the measurement unit;
a binding ratio calculation unit operable to calculate a binding ratio that is a ratio of FRET occurring receptors to the receptors contained in the measurement sample by using the fluorescence lifetime calculated by the fluorescence lifetime calculation unit; and
a dissociation constant calculation unit operable to calculate a dissociation constant indicating a degree of binding between the receptors and the ligands,
wherein the dissociation constant calculation unit determines the dissociation constant by using a least-squares method to fit a function to the binding ratio calculated by the binding ratio calculation unit under two or more binding conditions set so that the binding ratio is changed, the function having, as variables, a total concentration of the receptors in the measurement sample and the dissociation constant.

When the binding ratio, a total concentration of the receptors contained in the measurement sample, a total concentration of the ligands contained in the measurement sample, and the dissociation constant are defined as $\kappa_{FRET}$, $[R_0]$, $[L_0]$, and $K_d$, respectively, the dissociation constant calculation unit preferably determines the dissociation constant $K_d$ by using a two-variable least-squares method using the $[R_0]$ and the $K_d$ as variables to fit the following formula to the $\kappa_{FRET}$ and the $[L_0]$:

[Formula 7]

$$\kappa_{FRET} = \frac{[L_0] + [R_0] + K_d - \sqrt{([L_0] + [R_0] + K_d)^2 - 4[L_0][R_0]}}{2[R_0]}$$

The fluorescence lifetime calculation unit preferably calculates a fluorescence lifetime of the first molecule by using a phase difference between a fluorescence signal measured by the measurement unit and a modulation signal that modulates the laser light.

Preferably, the first molecule and the second molecule are bound to one of the receptors, and the dissociation constant calculation unit determines the dissociation constant by fitting the binding ratio calculated by the binding ratio calculation unit under two or more binding conditions set by adding ligands to the measurement sample so that the binding ratio is changed.

Alternatively, it is preferable that the first molecule is bound to one of the receptors, the second molecule is bound to one of the ligands, and the dissociation constant calculating unit determines the dissociation constant by fitting the binding ratio calculated by the binding ratio calculation unit under two or more binding conditions set by adding ligands to the measurement sample so that the binding ratio is changed.

Advantageous Effects of Invention

In the FRET measurement method and the FRET measurement device according to the present invention, a dissociation constant $K_d$ can be accurately measured irrespective of the magnitude of the dissociation constant $K_d$.

Brief Description of Drawings

Fig. 1 is a schematic diagram illustrating the configuration of a flow cytometer according to one embodiment of a FRET measurement device.

Fig. 2A is a diagram illustrating a sample in a state where a ligand is not bound to a receptor and Fig. 2B is a diagram illustrating the sample in a state where the ligand is bound to the receptor.

Fig. 3 is a diagram illustrating examples of an energy absorption spectrum and a fluorescence emission spectrum of a donor molecule and examples of an energy absorption spectrum and a fluorescence emission spectrum of an acceptor molecule.

Fig. 4 is a schematic diagram illustrating one example of the configuration of a measurement unit of the flow cytometer illustrated in Fig. 1.

Fig. 5 is a schematic diagram illustrating one example of the configuration of a control/processing unit of the flow cytometer illustrated in Fig. 1.

Fig. 6 is a schematic diagram illustrating one example of the configuration an analysis device of the flow cytometer illustrated in Fig. 1.

Fig. 7 is a flow chart illustrating one example of a FRET measurement method.

Fig. 8 is a graph illustrating one example of results of measurement of a fluorescence lifetime of the donor molecule relative to the total concentration of the ligand.

Fig. 9 is a graph illustrating one example of results of measurement of a binding ratio relative to the total concentration of the ligand.

Description of Embodiments

(Schematic Configuration of FRET Measurement Device)

Hereinbelow, a method and a device for measuring FRET according to the present invention will be described in detail.

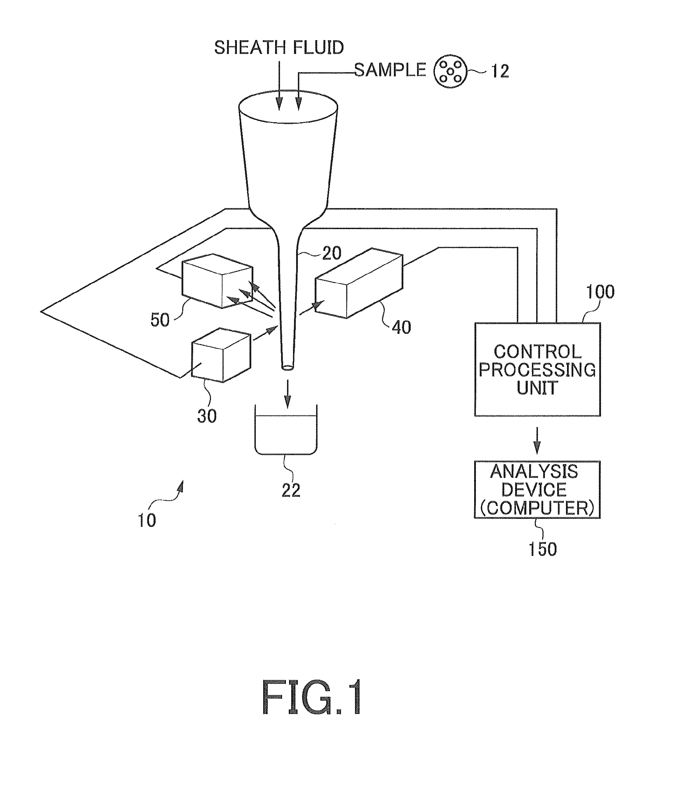

Fig. 1 is a schematic diagram illustrating the configuration of a flow cytometer 10 as one embodiment of the FRET measurement device according to the present invention.

The flow cytometer 10 according to this embodiment irradiates with laser light a sample 12 (measurement sample) obtained by, for example, labeling a protein in a living cell to be measured with donor and acceptor molecules, and measures fluorescence emitted by the sample 12. The flow cytometer 10 uses a measured fluorescence signal to determine the value of a dissociation constant $K_d$. As illustrated in Fig. 1, the flow cytometer 10 includes a tube 20, a laser light source unit 30, measurement units 40 and 50, a control/processing unit 100, and an analysis device 150.

The sample 12 flows through the tube 20 together with a sheath fluid forming a high-speed flow. A recovery container 22 that recovers the sample 12 is provided at the outlet of the tube 20.

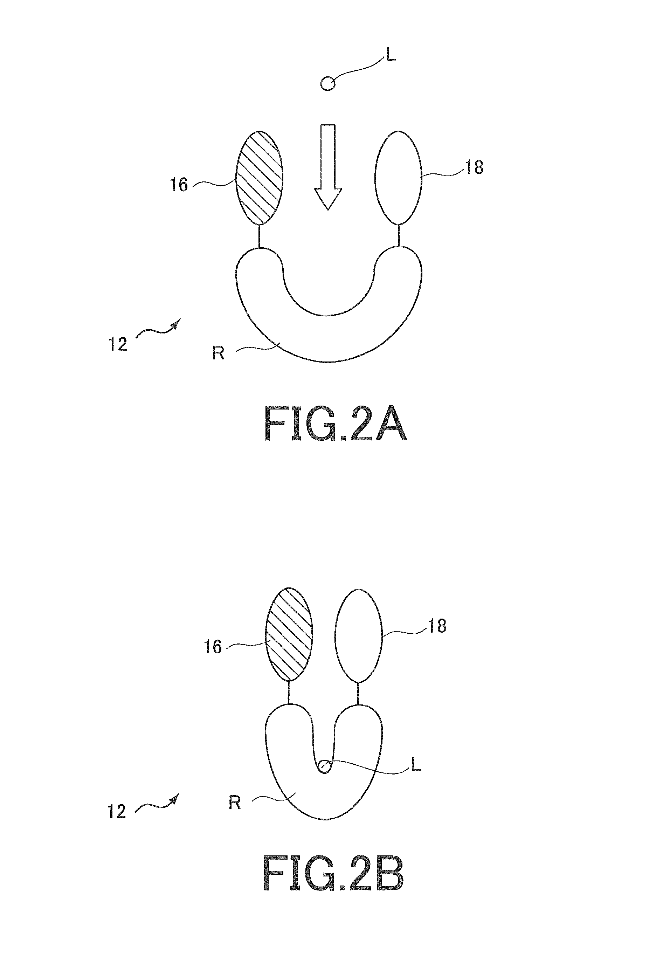

Here, the sample 12 according to this embodiment will be described with reference to Fig. 2. In the sample 12 according to this embodiment, a donor molecule 16 and an acceptor molecule 18 are bound to a receptor R. Fig. 2A is a diagram illustrating a sample 12 in a state where a ligand L is not bound to the receptor R and Fig. 2B is a diagram illustrating the sample 12 in a state where the ligand L is bound to the receptor R. As illustrated in Figs. 2A and 2B, according to this embodiment, the receptor R deforms when the ligand L binds to the receptor R. As a result, the distance between the donor molecule 16 and the acceptor molecule 18 is reduced so that a FRET phenomenon occurs. Therefore, it is possible to know the degree of binding of the ligand L to the receptor R by measuring a FRET phenomenon with the use of the flow cytometer 10 according to this embodiment.

Referring to Fig. 1 again, the laser light source unit 30 irradiates the sample 12 with laser light whose intensity is time-modulated. When the sample 12 is irradiated with laser light, the donor molecule 16 and the acceptor molecule 18 each absorb energy. For example, when the donor molecule 16 is CFP (Cyan Fluorescent Protein) and the acceptor molecule 18 is YFP (Yellow Fluorescent Protein), laser light having a wavelength of 405 to 450 nm at which the donor molecule 16 mainly absorbs energy is used. The laser light source unit 30 is, for example, a semiconductor laser. The output of laser light emitted by the laser light source unit 30 is, for example, 5 to 100 mW.

Hereinbelow, the relationship between the wavelength of laser light emitted by the laser light source unit 30 and the wavelength at which the donor molecule 16 and the acceptor molecule 18 absorb energy and the occurrence of FRET will be described.

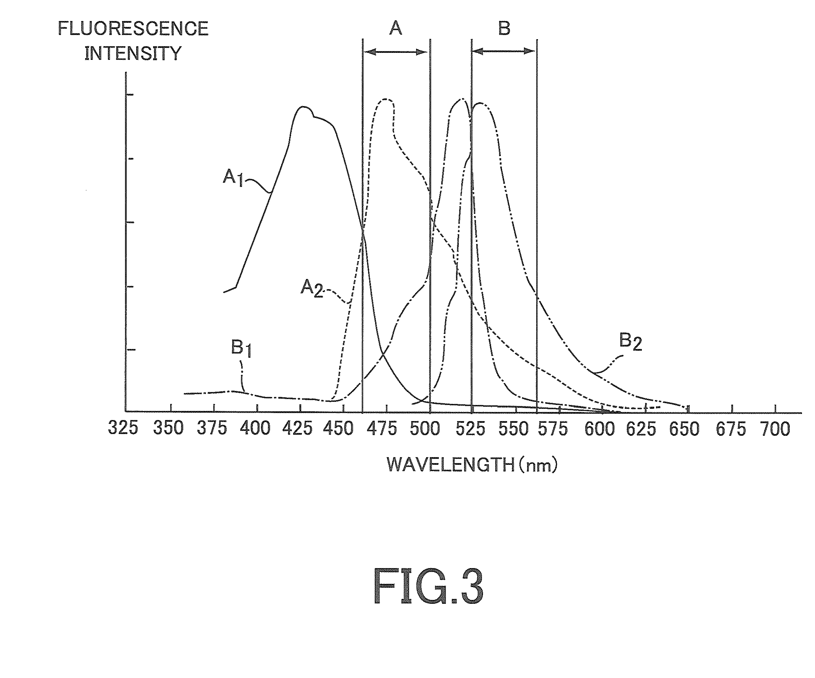

Fig. 3 is a diagram illustrating the energy absorption spectrum and fluorescence emission spectrum when the donor molecule 16 is CFP and the acceptor molecule 18 is YFP. More specifically, a curve $A_1$ is the energy absorption spectrum of the donor molecule 16, and a curve $A_2$ is the fluorescence emission spectrum of the donor molecule 16. Further, a curve $B_1$ is the energy absorption spectrum of the acceptor molecule 18, and a curve $B_2$ is the fluorescence emission spectrum of the acceptor molecule 18.

As illustrated in Fig. 3, the wavelength range in which the donor molecule 16 mainly absorbs energy is 405 to 450 nm. On the other hand, the wavelength range in which the acceptor molecule 18 mainly absorbs energy is 470 to 530 nm.

In general, when the distance between the donor molecule 16 and the acceptor molecule 18 is 10 nm or less, part of energy absorbed by the donor molecule 16 by irradiation with laser light is transferred to the acceptor molecule 18 by coulomb interaction. The acceptor molecule 18 absorbs the energy transferred from the donor molecule 16 by coulomb interaction, and is therefore excited to emit fluorescence. This phenomenon is called fluorescence resonance energy transfer (FRET).

FRET occurs also when CFP is used as the donor molecule 16 and YFP is used as the acceptor molecule 18. That is, energy is transferred from the donor molecule 16 to the acceptor molecule 18 by coulomb interaction so that fluorescence resulting from the excitation of the acceptor molecule 18 is emitted.

Referring to Fig. 1 again, the measurement unit 40 is arranged on the opposite side of the laser light source unit 30 across the tube 20. The measurement unit 40 includes a photoelectric converter that outputs a detection signal indicating the passage of the sample 12 through a measurement point by detecting laser light forward-scattered by the sample 12 which passes through the measurement point. The signal outputted by the measurement unit 40 is supplied to the control/processing unit 100. The signal supplied from the measurement unit 40 to the control/processing unit 100 is used as a trigger signal to announce the timing of passage of the sample 12 through the measurement point in the tube 20.

The measurement unit 50 is arranged on the line of intersection of a plane that passes through the measurement point and is perpendicular to a direction in which laser light emitted from the laser light source unit 30 travels and a plane that passes through the measurement point and is perpendicular to a direction in which the sample 12 moves in the tube 20. The measurement unit 50 includes a photoelectric converter that receives fluorescence emitted by the sample 12 irradiated with laser light at the measurement point. The photoelectric converter is, for example, a photomultiplier or an avalanche photodiode.

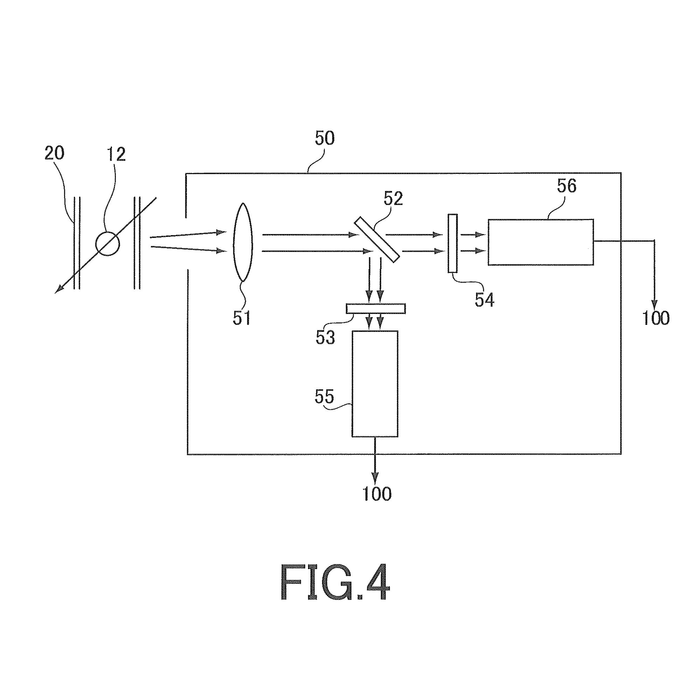

Hereinbelow, the configuration of the measurement unit 50 will be described in detail with reference to Fig. 4. Fig. 4 is a schematic diagram illustrating one example of the configuration of the measurement unit 50 according to this embodiment. As illustrated in Fig. 4, the measurement unit 50 includes a lens system 51, a dichroic mirror 52, band-pass filters 53 and 54, and photoelectric converters 55 and 56.

The lens system 51 focuses fluorescence emitted by the sample 12. The transmission and reflection wavelength characteristics of the dichroic mirror 52 are set so that the dichroic mirror 52 transmits fluorescence emitted by the acceptor molecule 18 and reflects fluorescence emitted by the donor molecule 16.

The band-pass filters 53 and 54 are provided in front of the light-receiving surfaces of the photoelectric converters 55 and 56, respectively. Each of the band-pass filters 53 and 54 transmits only fluorescence within a predetermined wavelength band. More specifically, the band-pass filter 53 is set so as to transmit fluorescence within a wavelength band (indicated by A in Fig. 3) in which fluorescence is emitted mainly by the donor molecule 16. On the other hand, the band-pass filter 54 is set so as to transmit fluorescence within a wavelength band (indicated by B in Fig. 3) in which fluorescence is emitted mainly by the acceptor molecule 18.

Each of the photoelectric converters 55 and 56 converts received light into an electric signal. The photoelectric converters 55 and 56 are, for example, sensors equipped with a photomultiplier. Fluorescence received by the photoelectric converters 55 and 56 has a phase delayed from the intensity-modulated laser light. Therefore, each of the photoelectric converters 55 and 56 receives an optical signal having information about a phase difference with respect to the intensity-modulated laser light and converts the optical signal into an electric signal. The signal (fluorescence signal) outputted from each of the photoelectric converters 55 and 56 is supplied to the control/processing unit 100.

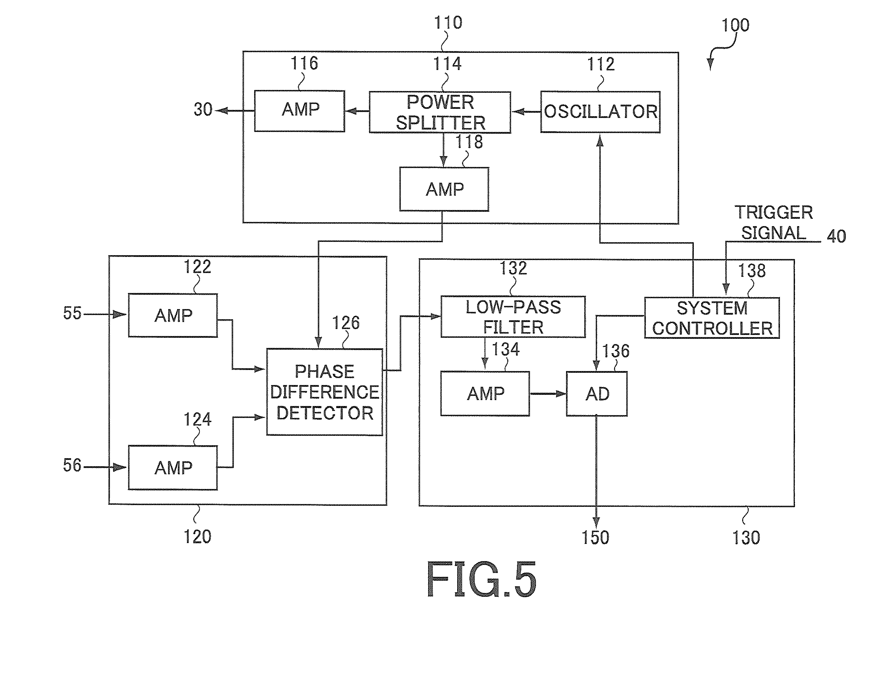

Hereinbelow, the configuration of the control/processing unit 100 will be described in detail with reference to Fig. 5. Fig. 5 is a schematic diagram illustrating one example of the configuration the control/processing unit 100 according to this embodiment. As illustrated in Fig. 5, the control/processing unit 100 includes a signal generation section 110, a signal processing section 120, and a controller 130.

The signal generation section 110 generates a modulation signal for time-modulating the intensity of laser light. The modulation signal is, for example, a sinusoidal signal having a predetermined frequency. The frequency is set to a value in the range of 10 MHz to 100 MHz.

The signal generation section 110 includes an oscillator 112, a power splitter 114, and amplifiers 116 and 118. The modulation signal generated by the oscillator 112 is split by the power splitter 114 and supplied to the laser light source unit 30 and the signal processing section 120. As will be described later, the signal generation section 110 supplies the modulation signal to the signal processing section 120 so as to use the modulation signal as a reference signal for measuring the phase difference of the fluorescence signal with respect to the modulation signal. Further, the modulation signal is used as a signal for modulating the amplitude of laser light emitted by the laser light source unit 30.

The signal processing section 120 extracts information about fluorescence emitted by the sample 12 with the use of the fluorescence signals outputted from the photoelectric converters 55 and 56. The information about fluorescence emitted by the sample 12 is, for example, information about fluorescence intensity or information about fluorescence lifetime. The signal processing section 120 includes amplifiers 122 and 124 and a phase difference detector 126.

The amplifiers 122 and 124 amplify the signals outputted from the photoelectric converters 55 and 56 and output the amplified signals to the phase difference detector 126.

The phase difference detector 126 detects the phase difference of each of the fluorescence signals outputted from the photoelectric converters 55 and 56 with respect to the modulation signal (reference signal). The phase difference detector 126 includes an IQ mixer (not illustrated). The IQ mixer multiplies the reference signal and the fluorescence signal to calculate a processing signal including a cos component (real part) of the fluorescence signal and a high-frequency component. Further, the IQ mixer multiplies a signal obtained by shifting the phase of the reference signal by 90 degrees and the fluorescence signal to calculate a processing signal including a sin component (imaginary part) of the fluorescence signal and a high-frequency component.

The controller 130 controls the signal generation section 110 so that the signal generation section 110 generates a sinusoidal signal having a predetermined frequency. Further, the controller 130 removes the high-frequency component from the processing signal outputted from the signal processing section 120 and including the cos component of the fluorescence signal and from the processing signal outputted from the signal processing section 120 and including the sin component of the fluorescence signal to determine the cos component and sin component of the fluorescence signal.

The controller 130 includes a low-pass filter 132, an amplifier 134, an A/D converter 136, and a system controller 138. The low-pass filter 132 removes the high-frequency component from the signals outputted from the signal processing section 120, the signals including the cos component or sin component of the fluorescence signal and the high-frequency component. The amplifier 134 amplifies the processing signal of cos component of the fluorescence signal and the processing signal of sin component of the fluorescence signal, which are signals obtained by removing the high-frequency component by the low-pass filter 132. The amplifier 134 outputs the amplified processing signals to the A/D converter 136. The A/D converter 136 samples the processing signal of cos component of the fluorescence signal and the processing signal of sin component of the fluorescence signal and supplies the sampled processing signals to the analysis device 150. The system controller 138 receives the input of the trigger signal outputted from the measurement unit 40. Further, the system controller 138 controls the oscillator 112 and the A/D converter 136.

Referring to Fig. 1 again, the analysis device 150 calculates a fluorescence lifetime, a binding ratio, a dissociation constant etc. from the processing signal of cos component (real part) of the fluorescence signal and the processing signal of sin component (imaginary part) of the fluorescence signal.

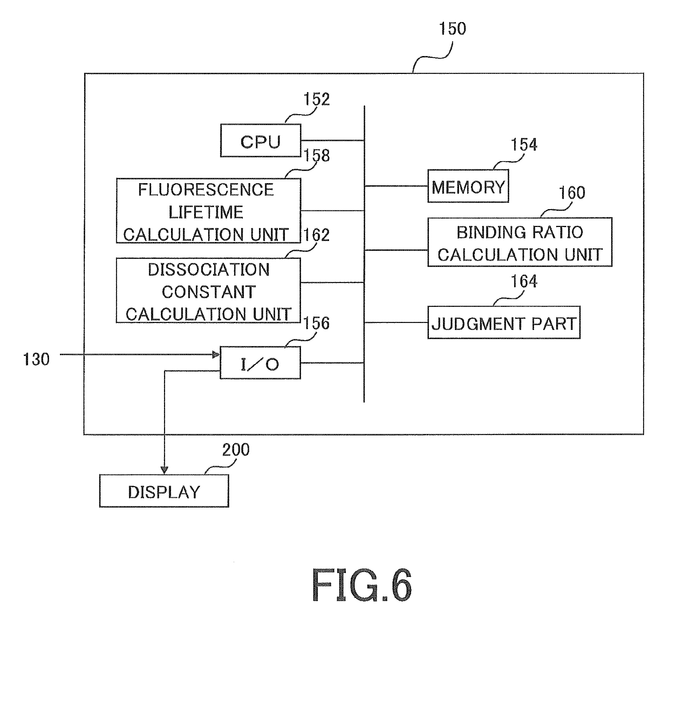

The analysis device 150 is a device configured by running a predetermined program on a computer. Hereinbelow, the configuration of the analysis device 150 will be described in detail with reference to Fig. 6. Fig. 6 is a schematic diagram illustrating the configuration of one example of the analysis device 150 according to this embodiment. As illustrated in Fig. 6, the analysis device 150 includes a CPU 152, a memory 154, an input/output port 156, a fluorescence lifetime calculation unit 158, a binding ratio calculation unit 160, a dissociation constant calculation unit 162, and a judgment part 164.

The analysis device 150 is connected with a display 200.

The CPU 152 is an arithmetic processor provided in the computer. The CPU 152 substantially executes various calculations required by the fluorescence lifetime calculation unit 158, the binding ratio calculation unit 160, and the dissociation constant calculation unit 162.

The memory 154 includes a ROM that stores the program executed on the computer to configure the fluorescence lifetime calculation unit 158, the binding ratio calculation unit 160, and the dissociation constant calculation unit 162 and a RAM that stores processing results calculated by these units and data supplied from the input/output port 156.

The input/output port 156 receives the input of values of the cos component (real part) and sin component (imaginary part) of the fluorescence signal supplied from the controller 130. Further, the input/output port 156 outputs processing results calculated by each of the units onto the display 200.

The display 200 displays various information and processing results determined by each of the units.

The fluorescence lifetime calculation unit 158 calculates the fluorescence lifetime of the donor molecule 16 with the use of the fluorescence signal measured by the measurement unit 50. For example, the fluorescence lifetime calculation unit 158 determines the phase difference of the fluorescence signal with respect to the modulation signal from the values of cos component and sin component of the fluorescence signal supplied from the controller 130. Further, the fluorescence lifetime calculation unit 158 calculates the fluorescence lifetime of the donor molecule 16 with the use of the obtained phase difference. More specifically, the fluorescence lifetime calculation unit 158 divides the tan component of the phase difference by the angular frequency of the modulation signal based on a formula (21) that will be described later to calculate the fluorescence lifetime. The fluorescence lifetime is expressed as a fluorescence relaxation time constant defined by assuming that the fluorescence components emitted by irradiation with laser light are based on a relaxed response of first-order lag system.

The binding ratio calculation unit 160 calculates a binding ratio, which is the ratio of the receptors R, in which FRET is occurring, to the receptors R contained in the sample 12, with the use of the fluorescence lifetime calculated by the fluorescence lifetime calculation unit 158. More specifically, the binding ratio calculation unit 160 calculates a binding ratio $\kappa_{FRET}$ defined by a formula (23) that will be described later.

The dissociation constant calculation unit 162 calculates a dissociation constant $K_d$ indicating the degree of binding between the receptor R and the ligand L. More specifically, the dissociation constant calculation unit 162 determines a dissociation constant $K_d$ by performing fitting to the binding ratios $\kappa_{FRET}$ calculated by the binding ratio calculation unit 160 with the use of a least-squares method using, as variables, the total concentration of the receptor R in the sample 12 and the dissociation constant $K_d$. Even more specifically, the dissociation constant calculation unit 162 determines a dissociation constant $K_d$ by performing fitting of a formula (8), which will be described later, with the use of a two-variable least-squares method using, as variables, the total concentration of the receptor R in the sample 12 and the dissociation constant $K_d$.

The judgment part 164 judges whether or not the dissociation constant calculation unit 162 has obtained a predetermined number of pieces of measured data required for performing fitting with the use of a two-variable least-squares method.

The above is the configuration of the flow cytometer 10 according to this embodiment.

(Summary of FRET Measurement Method)

Hereinbelow, a summary of the FRET measurement method according to the present invention will be described. As has been described above with reference to Fig. 2, according to this embodiment, a FRET phenomenon is caused by binding of the ligand L to the receptor R. In the sample 12, the receptor R and the ligand L reach a state of equilibrium represented by the above formula (1). Here, the following formula (7) is obtained by eliminating [L] and [R] from the above formulas (2), (3), and (4):

[Formula 8]

$$[LR]^2 - ([L_0] + [R_0] + K_d)[LR] + [L_0][R_0] = 0 \qquad (7)$$

The following formula (8) is obtained by solving the above formula (7) for [LR] under the condition that [LR] < [L_0]:

[Formula 9]

$$\frac{[LR]}{[R_0]} = \frac{[L_0] + [R_0] + K_d - \sqrt{([L_0] + [R_0] + K_d)^2 - 4[L_0][R_0]}}{2[R_0]} \qquad (8)$$

The above formula (8) is the exact solution of the formula (7), and is a relational expression that holds irrespective of the magnitude of the dissociation constant $K_d$. $[LR]/[R_0]$ represents the ratio of the receptors R bound to the ligands L to the total receptors R. The following description will be made assuming that FRET is always caused by binding of the ligand L to the receptor R. The ratio of the receptors R, in which FRET is occurring, to the receptors R contained in the sample 12, that is, $[LR]/[R_0]$ is defined as a binding ratio $\kappa_{FRET}$.

According to the FRET measurement method of the present invention, the binding ratio $\kappa_{FRET}$ is measured at different values of the total ligand concentration $[L_0]$ by changing the total ligand concentration $[L_0]$. Further, the dissociation constant $K_d$ is determined by fitting a curve that is based on the above formula (8) to the data of the total ligand concentration $[L_0]$ and the binding ratio $\kappa_{FRET}$ with the use of a two-variable least-squares method using, as variables, the total receptor concentration $[R_0]$ and the dissociation constant $K_d$.

(Method for Calculating Binding Ratio $\kappa_{FRET}$)

First, a method for calculating the binding ratio $\kappa_{FRET}$ will be described.

The total concentration of the receptor in the sample 12 is defined as $C_0$, the concentration of the receptor in which FRET is occurring is defined as $C^+$, and the concentration of the receptor in which no FRET is occurring is defined as $C^-$. Here, $C_0$, $C^+$, and $C^-$ satisfy the following equation: $C^- = C_0 - C^+$. Excitation power obtained by laser light is defined as P(t), the number of excited electrons of the donor molecule 16 bound to the receptor R in which FRET is occurring is defined as $N^+(t)$, and the number of excited electrons of the donor molecule 16 bound to the receptor R in which no FRET is occurring is defined as $N^-(t)$. At this time, rate equations for the excited electrons are represented by the following formulas (9) and (10):

[Formula 10]

$$\frac{dN^+(t)}{dt} = -(k_f + k_{nr} + k_t)N^+(t) + C^+ K P(t) \qquad (9)$$

$$\frac{dN^-(t)}{dt} = -(k_f + k_{nr})N^-(t) + C^- K P(t) \qquad (10)$$

wherein $k_f$ is the rate constant of radiative transition, $k_{nr}$ is the rate constant of non-radiative transition, $k_t$ is the rate constant of resonance energy transfer, and KP(t) is the number of electrons excited by laser light per unit time and per unit volume.

The binding ratio $\kappa_{FRET}$, that is, the ratio of the receptors R, in which FRET is occurring, to the receptors R contained in the sample 12 is represented by the following formula (11):

[Formula 11]

$$\kappa_{FRET} = \frac{C^+}{C_0} \quad (11)$$

Here, rate equations for the excited electrons are represented by the following formulas (12) and (13) by eliminating $C^+$ and $C^-$ from the formulas (9), (10), and (11) and by assuming that $K_{ex} = C_0 K$:

[Formula 12]

$$\frac{dN^+(t)}{dt} = -(k_f + k_{nr} + k_t)N^+(t) + \kappa_{FRET}K_{ex}P(t) \quad (12)$$

$$\frac{dN^-(t)}{dt} = -(k_f + k_{nr})N^-(t) + (1 - \kappa_{FRET})K_{ex}P(t) \quad (13)$$

The following formulas (14) and (15) are obtained by Laplace-transforming the formulas (12) and (13):

[Formula 13]

$$N^+(s) = \frac{\kappa_{FRET}K_{ex}}{s + k_f + k_{nr} + k_t}P(s) = \frac{\kappa_{FRET}K_{ex}\tau_{min}}{1 + \tau_{min}s}P(s) \quad (14)$$

$$N^-(s) = \frac{(1 - \kappa_{FRET})K_{ex}}{s + k_f + k_{nr}}P(s) = \frac{(1 - \kappa_{FRET})K_{ex}\tau_{max}}{1 + \tau_{max}s}P(s) \quad (15)$$

wherein $\tau_{min}$ is a minimum value of fluorescence lifetime of the donor molecule 16 and satisfies the following relational expression: $1/\tau_{min} = k_f + k_{nr} + k_t$ and $\tau_{max}$ is a maximum value of fluorescence lifetime of the donor molecule 16 and satisfies the following relational expression: $1/\tau_{max} = k_f + k_{nr}$.
$\tau_{min}$ is the fluorescence lifetime of the donor molecule 16 when FRET is occurring in all the donor molecules 16 in the sample 12. For example, $\tau_{min}$ can be measured by measuring the fluorescence lifetime of the donor molecule 16 under the condition that the concentration of the acceptor molecules 18 is made sufficiently higher than that of the donor molecules 16.
On the other hand, $\tau_{max}$ is the fluorescence lifetime of the donor molecule 16 when FRET is not occurring in any of the donor molecules 16 in the sample 12. For example, $\tau_{max}$ can be measured by measuring the fluorescence lifetime of the donor molecule 16 under the condition that the acceptor molecules 18 are not added to the sample 12.
In this way, the values of $\tau_{min}$ and $\tau_{max}$ can be determined by measurement.
The number of excited electrons N(s) of the donor molecule 16 in the sample 12 is represented by the following formula (16) based on the above formulas (14) and (15):

[Formula 14]

$$N(s) = N^+(s) + N^-(s)$$

$$= \left(\frac{\kappa_{FRET}\tau_{min}}{1 + \tau_{min}s} + \frac{(1 - \kappa_{FRET})\tau_{max}}{1 + \tau_{max}s}\right)K_{ex}P(s) \quad (16)$$

The part in parentheses in the formula (16) relates to the fluorescence lifetime of the donor molecule 16, and therefore when the part in parentheses in the formula (16) is defined as T, the following formula (17) is obtained by arranging the formula (16):

[Formula 15]

$$T = \frac{\tau_{max} - \kappa_{FRET}(\tau_{max} - \tau_{min}) + \tau_{max}\tau_{min}s}{1 + \tau_{min}\tau_{max}s^2 + (\tau_{min} + \tau_{max})s} \qquad (17)$$

Here, when $j\omega$ is substituted for s in the above formula (17), the following formula (18) is obtained by arranging the formula (17):

[Formula 16]

$$T = \sqrt{\frac{\{\tau_{max} - \kappa_{FRET}(\tau_{max} - \tau_{min})\}^2 + (\tau_{max}\tau_{min}\omega)^2}{(1 - \tau_{min}\tau_{max}\omega^2)^2 + \{(\tau_{min} + \tau_{max})\omega\}^2}}e^{j(\theta_1 - \theta_2)} \qquad (18)$$

Here, $\theta_1$ and $\theta_2$ are represented by the following formulas (19) and (20), respectively:

[Formula 17]

$$\theta_1 = \tan^{-1}\frac{\tau_{max}\tau_{min}\omega}{\tau_{max} - \kappa_{FRET}(\tau_{max} - \tau_{min})} \qquad (19)$$

$$\theta_2 = \tan^{-1}\frac{(\tau_{min} + \tau_{max})\omega}{1 - \tau_{min}\tau_{max}\omega^2} \qquad (20)$$

Here, $\omega$ is the angular frequency of the modulation signal that modulates laser light, and $\theta_1 - \theta_2$ is the phase difference between the fluorescence signal of the donor molecule 16 and the modulation signal that modulates laser light.

The fluorescence lifetime $\tau$ of the donor molecule 16 is determined by the following formula (21) from the phase difference between the fluorescence signal of the donor molecule 16 and the modulation signal that modulates laser light and the angular frequency of the modulation signal:

[Formula 18]

$$\tau = \frac{-\tan(\theta_1 - \theta_2)}{\omega} \qquad (21)$$

Here, the following formula (22) is obtained by eliminating $\theta_1$ from the above formulas (19) and (21):

[Formula 19]

$$\frac{\tau_{max}\tau_{min}\omega}{\tau_{max} - \kappa_{FRET}(\tau_{max} - \tau_{min})} = \tan\{\theta_2 - \tan^{-1}(\tau\omega)\} \qquad (22)$$

The following formula (23) is obtained by solving the above formula (22) for $\kappa_{FRET}$:

[Formula 20]

$$\kappa_{FRET} = \frac{\tau_{max} - \dfrac{\tau_{max}\tau_{min}\omega}{\tan\{\theta_2 - \tan^{-1}(\tau\omega)\}}}{\tau_{max} - \tau_{min}} \qquad (23)$$

The binding ratio $\kappa_{FRET}$ can be determined by the above formula (23) from the fluorescence lifetime $\tau$ of the donor molecule 16. It is to be noted that, as described above, the values of $\tau_{min}$ and $\tau_{max}$ can be separately determined by measurement. As will be described later, the association constant $K_d$ can be determined using the binding ratio $\kappa_{FRET}$ determined by the formula (23).

(FRET Measurement Method)

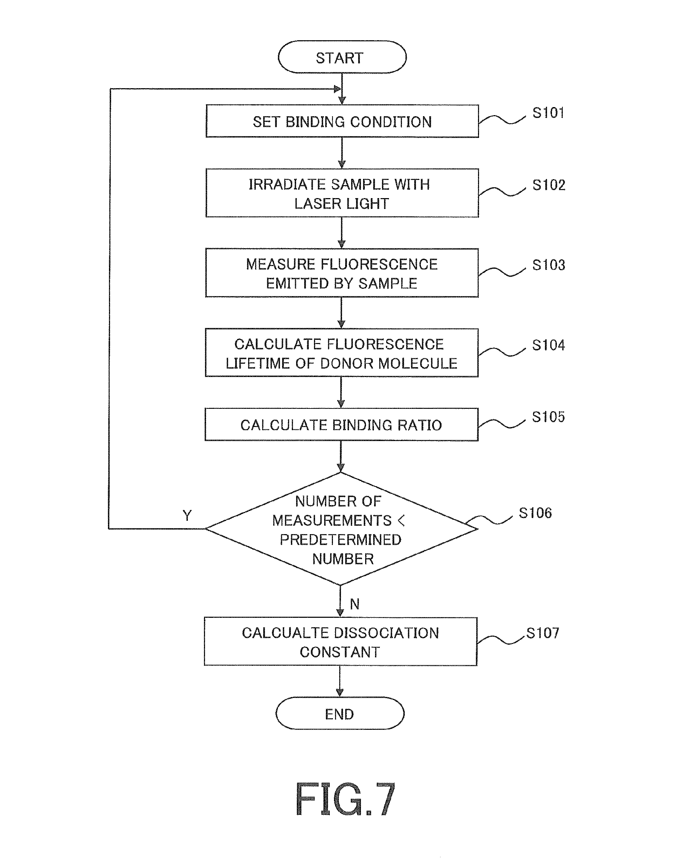

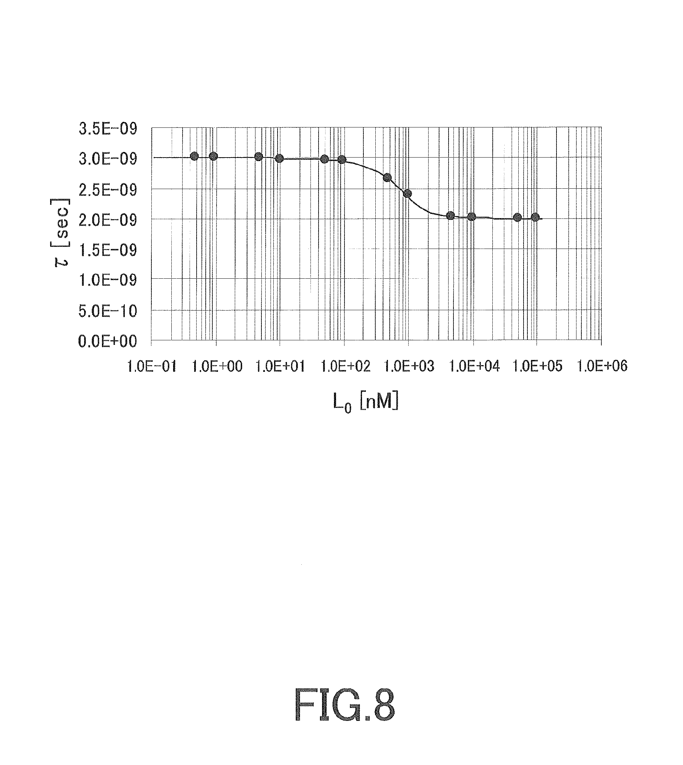

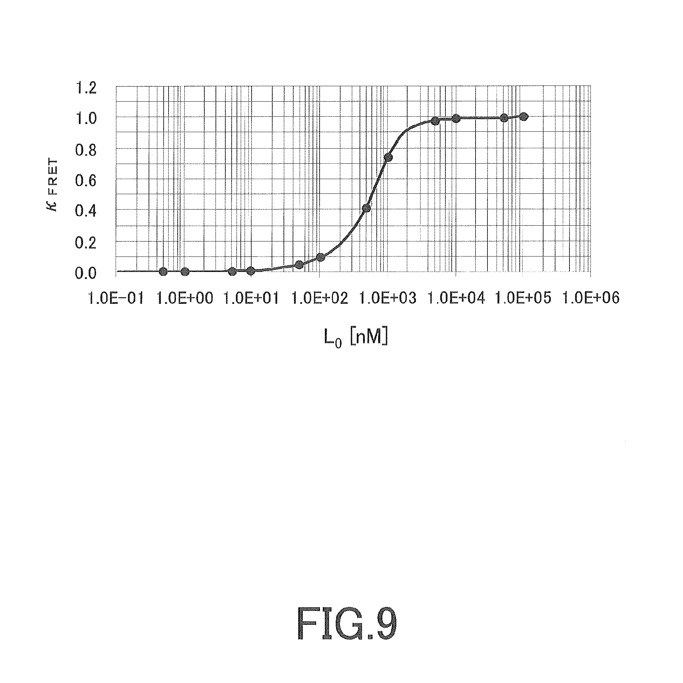

Hereinbelow, the FRET measurement method according to the present invention will be described with reference to Figs. 7, 8, and 9. Fig. 7 is a flow chart illustrating one example of the FRET measurement method according to the present invention. Fig. 8 is a diagram illustrating one example of the results of measurement of the fluorescence lifetime $\tau$ of the donor molecule 16 versus the total ligand concentration $[L_0]$. Fig. 9 is a diagram illustrating one example of the results of measurement of the binding ratio $\kappa_{FRET}$ versus the total ligand concentration $[L_0]$.

First, the flow cytometer 10 sets a binding condition for the sample 12 (Step S101). For example, the binding condition for the sample 12 is set by adjusting the total concentration $[L_0]$ of the ligand added to the sample 12. Then, the laser light source unit 30 irradiates the sample 12 with laser light whose intensity is time-modulated (Step S102). Then, the measurement unit 50 receives fluorescence emitted by the sample 12 irradiated with laser light at the measurement point (Step S103).

Then, the fluorescence lifetime calculation unit 158 calculates the fluorescence lifetime of the donor molecule 16 with the use of the fluorescence signal measured by the measurement unit 50 (Step S104). More specifically, the fluorescence lifetime calculation unit 158 calculates the fluorescence lifetime of the donor molecule 16 based on the above formula (21) from the phase difference ($\theta_1 - \theta_2$) between the fluorescence signal and the modulation signal. By performing the step S104, one of the measurement results illustrated in Fig. 8 is obtained.

Then, the binding ratio calculation unit 160 calculates the binding ratio $\kappa_{FRET}$ with the use of the fluorescence lifetime $\tau$ calculated by the fluorescence lifetime calculation unit 158 (Step S105). More specifically, the binding ratio calculation unit 160 calculates the binding ratio $\kappa_{FRET}$ based on the above formula (23) with the use of the fluorescence lifetime $\tau$ calculated by the fluorescence lifetime calculation unit 158. The binding ratio $\kappa_{FRET}$ is obtained according to the adjusted total ligand concentration $[L_0]$. Therefore, one of the measurement results illustrated in Fig. 9 is obtained by performing Step S105.

Then, the judgment part 164 judges whether or not a predetermined number of pieces of measured data required for performing fitting using a two-variable least-squares method has been obtained (Step S106). For example, about 10 to 15 pieces of measured data are preferably obtained to allow the dissociation constant calculation unit 162 to perform fitting using a two-variable least-squares method. When the judgment part 164 judges that the number of pieces of measured data obtained (the number of measurements) is less than a predetermined number, the above-described steps S101 to S105 are repeated until a predetermined number of pieces of measured data are obtained.

When the judgment part 164 judges that a predetermined number of pieces of measured data has been obtained, that is, the number of measurements is equal to a predetermined number, the dissociation constant calculation unit 162 calculates the dissociation constant $K_d$ indicating the degree of binding between the receptor R and the ligand L. More specifically, the dissociation constant calculation unit 162 performs fitting of the above formula (8) to the binding ratio $\kappa_{FRET}$ and the total ligand concentration $[L_0]$ with the use of a two-variable least-squares method using, as variables, the total receptor concentration $[R_0]$ and the dissociation constant $K_d$ to determine the dissociation constant $K_d$.

As has been described above, according to the present invention, the dissociation constant $K_d$ can be determined by using a least-squares method to fit a function to the binding ratio $\kappa_{FRET}$ and the total ligand concentration $[L_0]$, the function having, as variables, the total receptor concentration $[R_0]$ in the sample 12 and the dissociation constant $K_d$. Therefore, according to the present invention, it is possible to accurately measure the dissociation constant $K_d$ irrespective of the magnitude of the dissociation constant $K_d$.

It is to be noted that the above embodiments have been described with reference to a case where the donor molecule 16 and the acceptor molecule 18 are bound to the receptor R as described with reference to Fig. 2, but the present invention is not limited thereto. For example, the present invention can be applied also to a case where the donor molecule 16 is bound to the receptor R and the acceptor molecule 18 is bound to the ligand L.

The FRET measurement method and the FRET measurement device according to the present invention have been described above in detail, but the present invention is not limited to the above embodiments. It will be understood that various changes and modifications may be made without departing from the scope of the present invention.

Reference Signs List

| | |
|---|---|
| 10 | flow cytometer |
| 12 | sample |
| 16 | donor molecule |
| 18 | acceptor molecule |
| 20 | tube |
| 22 | recovery container |
| 30 | laser light source unit |
| 40, 50 | measurement unit |
| 51 | lens system |
| 52 | dichroic mirror |
| 53, 54 | band-pass filter |
| 55, 56 | photoelectric converter |
| 100 | control/processing unit |
| 110 | signal generation section |
| 112 | oscillator |
| 114 | power splitter |
| 116, 118 | amplifier |
| 120 | signal processing section |
| 122, 124 | amplifier |
| 126 | phase difference detector |
| 130 | controller |
| 132 | low-pass filter |
| 134 | amplifier |
| 136 | A/D converter |
| 138 | system controller |
| 150 | analysis device |
| 152 | CPU |
| 154 | memory |
| 156 | input/output port |
| 158 | fluorescence lifetime calculation unit |
| 160 | binding ratio calculation unit |
| 162 | dissociation constant calculation unit |
| 164 | judgment part |
| 200 | display |
| R | receptor |
| L | ligand |

**Claims**

1. A FRET measurement method for measuring, by irradiating a measurement sample with laser light, fluorescence resonance energy transfer (FRET) that is transfer of energy from a first molecule to a second molecule, the first molecule and the second molecule included in the measurement sample in which ligands are bound to receptors, the method comprising the steps of:

irradiating the measurement sample with laser light whose intensity is time-modulated;
measuring fluorescence emitted by the measurement sample irradiated with the laser light;
calculating a fluorescence lifetime of the first molecule by using a fluorescence signal measured in the measuring step;

calculating a binding ratio that is a ratio of FRET occurring receptors to the receptors contained in the measurement sample by using the fluorescence lifetime calculated in the fluorescence lifetime calculating step; setting a binding condition for the measurement sample so that the binding ratio is changed; and calculating a dissociation constant indicating a degree of binding between the receptors and the ligands, wherein, in the dissociation constant calculating step, the dissociation constant is determined by using a least-squares method to fit a function to the binding ratio calculated in the binding ratio calculating step, the function having, as variables, a total concentration of the receptors in the measurement sample and the dissociation constant.

2. The FRET measurement method according to claim 1, wherein when the binding ratio, a total concentration of the receptors contained in the measurement sample, a total concentration of the ligands contained in the measurement sample, and the dissociation constant are defined as $\kappa_{FRET}$, $[R_0]$, $[L_0]$, and $K_d$, respectively, the dissociation constant $K_d$ is determined in the dissociation constant calculating step by using a two-variable least-squares method using the $[R_0]$ and the $K_d$ as variables to fit the following formula to the $\kappa_{FRET}$ and the $[L_0]$:

[Formula 1]

$$\kappa_{FRET} = \frac{[L_0] + [R_0] + K_d - \sqrt{([L_0] + [R_0] + K_d)^2 - 4[L_0][R_0]}}{2[R_0]}$$

3. The FRET measurement method according to claim 1 or 2, wherein in the fluorescence lifetime calculating step, a fluorescence lifetime of the first molecule is calculated using a phase difference between a fluorescence signal measured in the measuring step and a modulation signal that modulates the laser light.

4. The FRET measurement method according to any one of claims 1 to 3, wherein the first molecule and the second molecule are bound to one of the receptors, and, in the binding condition setting step, a binding condition for the measurement sample is set by adding ligands to the measurement sample so that the binding ratio is changed.

5. The FRET measurement method according to any one of claims 1 to 3, wherein the first molecule is bound to one of the receptors, the second molecule is bound to one of the ligands, and, in the binding condition setting step, a binding condition for the measurement sample is set by adding ligands to the measurement sample so that the binding ratio is changed.

6. A FRET measurement device for measuring, by irradiating a measurement sample with laser light, fluorescence resonance energy transfer (FRET) that is transfer of energy from a first molecule to a second molecule, the first molecule and the second molecule included in the measurement sample in which ligands are bound to receptors, the device comprising:

a laser light source unit operable to irradiate the measurement sample with laser light whose intensity is time-modulated;
a measurement unit operable to measure fluorescence emitted by the measurement sample irradiated with the laser light;
a fluorescence lifetime calculation unit operable to calculate a fluorescence lifetime of the first molecule by using a fluorescence signal measured by the measurement unit;
a binding ratio calculation unit operable to calculate a binding ratio that is a ratio of FRET occurring receptors to the receptors contained in the measurement sample by using the fluorescence lifetime calculated by the fluorescence lifetime calculation unit; and
a dissociation constant calculation unit operable to calculate a dissociation constant indicating a degree of binding between the receptors and the ligands,
wherein the dissociation constant calculation unit is operable to determine the dissociation constant by using a least-squares method to fit a function to the binding ratio calculated by the binding ratio calculation unit under two or more binding conditions set so that the binding ratio is changed, the function having, as variables, a total concentration of the receptors in the measurement sample and the dissociation constant.

7. The FRET measurement device according to claim 6, wherein when the binding ratio, a total concentration of the receptors contained in the measurement sample, a total concentration of the ligands contained in the measurement sample, and the dissociation constant are defined as $\kappa_{FRET}$, $[R_0]$, $[L_0]$, and $K_d$, respectively, the dissociation constant calculation unit is operable to determine the dissociation constant $K_d$ by using a two-variable least-squares method using the $[R_0]$ and the $K_d$ as variables to fit the following formula to the $\kappa_{FRET}$ and the $[L_0]$:

[Formula 2]

$$\kappa_{FRET} = \frac{[L_0] + [R_0] + K_d - \sqrt{([L_0] + [R_0] + K_d)^2 - 4[L_0][R_0]}}{2[R_0]}$$

8. The FRET measurement device according to claim 6 or 7, wherein the fluorescence lifetime calculation unit is operable to calculate a fluorescence lifetime of the first molecule by using a phase difference between a fluorescence signal measured by the measurement unit and a modulation signal for modulating the laser light.

9. The FRET measurement device according to any one of claims 6 to 8, wherein the first molecule and the second molecule are bound to one of the receptors, and the dissociation constant calculation unit is operable to determine the dissociation constant by fitting the binding ratio calculated by the binding ratio calculation unit under two or more binding conditions set by adding ligands to the measurement sample so that the binding ratio is changed.

10. The FRET measurement device according to any one of claims 6 to 8, wherein the first molecule is bound to one of the receptors, the second molecule is bound to one of the ligands, and the dissociation constant calculating unit is operable to determine the dissociation constant by fitting the binding ratio calculated by the binding ratio calculation unit under two or more binding conditions set by adding ligands to the measurement sample so that the binding ratio is changed.

**FIG.1**

L

16

18

12

R

# FIG.2A

16

18

L

12

R

# FIG.2B

FIG.3

EP 2 570 798 A1

FIG.4

FIG.5

100

110

TRIGGER SIGNAL 40

112 OSCILLATOR

114 POWER SPLITTER

118 AMP

116 AMP → 30

138 SYSTEM CONTROLLER

130

132 LOW-PASS FILTER

134 AMP

136 AD → 150

126 PHASE DIFFERENCE DETECTOR

122 AMP ← 55

124 AMP ← 56

120

FIG.6

START

SET BINDING CONDITION — S101

IRRADIATE SAMPLE WITH
LASER LIGHT — S102

MEASURE FLUORESCENCE
EMITTED BY SAMPLE — S103

CALCULATE FLUORESCENCE
LIFETIME OF DONOR MOLECULE — S104

CALCULATE BINDING RATIO — S105

Y — NUMBER OF
MEASUREMENTS <
PREDETERMINED
NUMBER — S106

N

CALCUALTE DISSOCIATION
CONSTANT — S107

END

# FIG.7

FIG.8

FIG.9

| | INTERNATIONAL SEARCH REPORT | International application No. |
|---|---|---|
| | | PCT/JP2011/002546 |

A. CLASSIFICATION OF SUBJECT MATTER
*G01N21/64*(2006.01)i, *G01N33/542*(2006.01)i, *G01N33/566*(2006.01)i

According to International Patent Classification (IPC) or to both national classification and IPC

B. FIELDS SEARCHED

Minimum documentation searched (classification system followed by classification symbols)
G01N21/62-21/74, G01N33/48-33/98

Documentation searched other than minimum documentation to the extent that such documents are included in the fields searched
Jitsuyo Shinan Koho          1922-1996   Jitsuyo Shinan Toroku Koho   1996-2011
Kokai Jitsuyo Shinan Koho    1971-2011   Toroku Jitsuyo Shinan Koho   1994-2011

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2008/078526 A1  (Olympus Corp.),<br>03 July 2008 (03.07.2008),<br>paragraphs [0039] to [0047]; fig. 3, 4<br>& JP 2010-57364 A | 1,3-6,8-10<br>2,7 |
| Y<br>A | JP 2007-240424 A  (Mitsui Engineering &<br>Shipbuilding Co., Ltd.),<br>20 September 2007 (20.09.2007),<br>paragraphs [0058], [0080] to [0084], [0088] to<br>[0093]<br>(Family: none) | 1,3-6,8-10<br>2,7 |
| Y<br>A | JP 2008-514955 A  (Singulex, Inc.),<br>08 May 2008 (08.05.2008),<br>paragraphs [0754] to [0757]; fig. 22<br>& US 2006/0078998 A1     & EP 1805500 A<br>& WO 2006/036182 A2     & CN 101115985 A | 1,3-6,8-10<br>2,7 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 July, 2011 (22.07.11) | Date of mailing of the international search report<br>02 August, 2011 (02.08.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2011/002546 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2008-505321 A (Blueshift Biotechnologies, Inc.), 21 February 2008 (21.02.2008), entire text; all drawings & US 2006/0003320 A1 & EP 1774024 A & WO 2006/014351 A2 | 1-10 |
| A | JP 2005-513439 A (POWDERJECT RESEARCH LTD.), 12 May 2005 (12.05.2005), entire text; all drawings & US 2003/0113934 A1 & EP 1468281 A & WO 2003/052413 A1 & CA 2470820 A & AU 2002357002 A | 1-10 |
| A | JP 2003-075443 A (Sekisui Chemical Co., Ltd.), 12 March 2003 (12.03.2003), entire text; all drawings (Family: none) | 1-10 |
| A | JP 2003-503707 A (Ingeneus Corp.), 28 January 2003 (28.01.2003), entire text; all drawings & US 6645733 B1 & EP 1196776 A & WO 2001/001138 A1 & DE 60030291 D & AU 5098500 A & CA 2377815 A & HK 1046955 A & AU 769201 B & AT 337553 T & TW 266052 B | 1-10 |
| A | WO 2009/028062 A1 (Mitsui Engineering & Shipbuilding Co., Ltd.), 05 March 2009 (05.03.2009), entire text; all drawings & US 2010/0312482 A & EP 2196794 A1 & CN 101688836 A | 1-10 |
| A | Shigeyuki NAKATA et al., "Development of fluorescence lifetime FRET flow cytometer", Mitsui Zosen Technical Review, 31 March 2007 (31.03.2007), no.190, pages 54 to 60 | 1-10 |
| A | Li E, et.al., Quantitative Measurements of Protein Interactionsin a Crowded Cellular Environment, Analytical Chemistry, 2008.08.01, Vol. 80, No. 15, pp.5976-5985 | 1-10 |

Form PCT/ISA/210 (continuation of second sheet) (July 2009)

**EP 2 570 798 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007240424 A